Europäisches Patentamt

(19) European Patent Office

Office européen des brevets

(11) Veröffentlichungsnummer: **0 019 305**
**B1**

(12) **EUROPÄISCHE PATENTSCHRIFT**

(45) Veröffentlichungstag der Patentschrift:
**27.10.82**

(51) Int. Cl.³: **C 07 C 39/07**, C 07 C 39/04,
C 07 C 37/72

(21) Anmeldenummer: **80200084.4**

(22) Anmeldetag: **31.01.80**

(54) **Verfahren zur Aufbereitung von Phenolatlaugen.**

(30) Priorität: **12.05.79 DE 2919298**

(43) Veröffentlichungstag der Anmeldung:
**26.11.80 Patentblatt 80/24**

(45) Bekanntmachung des Hinweises auf die Patenterteilung:
**27.10.82 Patentblatt 82/43**

(84) Benannte Vertragsstaaten:
**DE FR GB IT NL**

(56) Entgegenhaltungen:
**DE-A-2 501 376**
**FR-A-1 061 870**
**US-A-1 944 681**
**US-A-2 037 295**
**US-A-2 137 587**
**US-A-2 807 654**

(73) Patentinhaber: **Rütgerswerke Aktiengesellschaft,
Mainzer Landstrasse 217, D-6000 Frankfurt
a.Main 1 (DE)**

(72) Erfinder: **Spengler, Hans, Dr., Holtmannstrasse 12,
D-4716 Olfen (DE)**
Erfinder: **Pachaly, Detlev, Dr., Natland 5, D-4330 Mülheim
(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Aufbereitung von Phenolatlaugen

Die vorliegende Erfindung bezieht sich auf ein Verfahren zur kontinuierlichen Aufbereitung von durch Fällung mit Kohlendioxid aus Natriumphenolat erzeugtem Rohphenol.

Phenol, Kresole und Xylenole (Phenole) aus Teer werden auf folgendem Weg gewonnen: In einer Primärdestillation werden die Phenole in der Leichtöl- und Karbolölfraktion angereichert; aus dem Karbolöl/Leichtöl werden die Phenole durch Extraktion mit Natronlauge unter Phenolatbildung abgetrennt. Aus Phenolat werden die Phenole entweder durch Zugabe von Schwefelsäure oder durch Begasen mit Kohlendioxid gefällt.

Bei den klassischen Fällungsprozessen mit Schwefelsäure bzw. Kohlendioxid entstehen Umweltgifte. Bei der Schwefelsäurefällung entsteht eine umweltbelastende phenolhaltige Natriumsulfatlösung. Bei der Kohlendioxidfällung entsteht phenolhaltige Sodalösung. Die Kohlendioxidfällung ist gewöhnlich mit einem Kalkbrenn- und Kaustifizierungsprozeß gekoppelt, um beim Kalkbrennen Kohlendioxid für die Fällung und im Kaustifizierungsprozeß Branntkalk mit der bei der Fällung angefallenen Sodalauge zu regenerierter Natronlauge und Kalkschlamm umzusetzen. Der erzeugte Kalkschlamm enthält Phenol und ist daher toxisch und umweltschädigend.

Die Fällung mit Kohlendioxid ist wirtschaftlicher als die Schwefelsäurefällung und wird meistens bevorzugt.

Ein derartiges Verfahren beschreibt die US-PS 1 944 681. Die durch Umsetzung mit Kohlendioxid freigesetzten Rohphenole sind mit der Sodalauge nicht mischbar und werden abgetrennt. Sie enthalten noch Salze, die ausgewaschen werden müssen. Das anfallende Waschwasser wird der Sodalauge wieder zugeschlagen.

Derartige Prozesse haben verschiedene Nachteile. So erfolgt beim Waschprozeß eine Phasenumkehr, wodurch der Prozeß nicht kontinuierlich gestaltet werden kann. Nach der Wasserwäsche sind im Rohphenol noch so viele Salze (0,5%) enthalten, daß in der nachfolgenden destillativen Aufarbeitung der Destillationssumpf nicht ausdestilliert werden kann, und das anfallende Sumpfprodukt (Phenolpech) einer nochmaligen Wasserwäsche zugeführt werden muß.

Auch wenn das Waschwasser der Sodalauge wieder zugeschlagen wird, verbleibt nach der Rohphenolabtrennung ein stark mit Phenolen kontaminiertes Wasser.

Die im gewaschenen Phenol verbleibenden Wassermengen führen in den Verdampferanlagen zu hohen zusätzlichen Energiekosten.

Aus der FR-PS 1 061 870 ist ein Verfahren bekannt, bei dem Phenolatlauge so lange mit Kohlendioxid begast wird, bis sich eine Rohphenolphase und eine Bicarbonatlauge gebildet haben. Die Rohphenolphase wird dann, gegebenenfalls im Gemisch mit Bicarbonatlauge oder einer anderen Salzlösung, mit einem Lösungsmittel extrahiert und die Bicarbonatlauge, sofern sie nicht gemeinsam mit Rohphenol extrahiert wurde, wird anschließend mit phenolhaltigem Lösungsmittel behandelt.

Das Verfahren hat die Nachteile, daß ein hoher Bicarbonatanteil entsteht, der bei der Aufarbeitung zu einem hohen Branntkalkverbrauch und somit auch zu einem hohen Anfall von Kalkschlamm führt. Außerdem enthalten die Lösungsmittelextrakte noch relativ große Mengen an Wasser samt den darin gelösten Salzen. Diese Salze bedingen bei der destillativen Aufarbeitung der Lösungsmittelextrakte eine relativ starke Pechbildung.

Der Erfindung lag deshalb die Aufgabe zugrunde, die geschilderten Nachteile durch eine komplexe Verfahrensänderung zu eliminieren. Es sollte ein Verfahren zur Aufbereitung der aus der Fällung mit Kohlendioxid stammenden Rohphenole gefunden werden, das kontinuierlich zu fahren ist und gleichzeitig die Entstehung von kontaminierenden Abfallstoffen vermindert.

Die gestellte Aufgabe wird erfindungsgemäß dadurch gelöst, daß kontinuierlich der phenolhaltige Lösungsmittelextrakt aus der Sodalaugeextraktion mit Rohphenol gemischt, das Gemisch mit Wasser im Gegenstrom gewaschen und das Waschwasser, das Phenolat, Soda und Bicarbonat enthält, zur phenolhaltigen Sodalauge zurückgegeben wird.

Als Lösungsmittel kommen solche in Frage, die nach ihrer destillativen Abtrennung ein neutralölfreies Rohphenol zurücklassen. Es eignen sich z. B. Benzol, Toluol, Cyclohexan und besonders Diisopropyläther, dessen hohes Extraktionsvermögen gegenüber Phenolen aus Wasser an sich bekannt ist.

Die Vorteile der getroffenen Maßnahmen bestehen darin, daß durch Extraktion der Sodalauge mit dem Lösemittel verhindert wird, daß der nachfolgend produzierte Kalkschlamm Phenole enthält. Darüber hinaus kann die Extraktion des gefällten Phenols mit Wasser kontinuierlich erfolgen, weil die organische Phase durch den Ätherzusatz immer leichter ist als die wäßrige Phase, so daß keine Phasenumkehr mehr erfolgen kann. Da alles Waschwasser in die Sodalauge gelangt, die entphenolt wird, entsteht kein kontaminiertes Wasser mehr. Das mit Wasser gewaschene Gemisch aus Phenolen und Lösungsmittel enthält so wenige Salze, daß der Destillationssumpf voll ausdestilliert werden kann.

Das nachfolgende Beispiel erläutert das erfindungsgemäße Verfahren.

### Beispiel

750 kg Phenolatlauge (24% gebundenes Rohphenol) werden auf bekannte Weise mit $CO_2$ begast, bis sich eine organische und eine wäßrige Phase gebildet haben.

Die wäßrige Phase enthält 16—20% Soda, 1—2% Bicarbonat und 2 bis 4% Phenole.

Die organische Phase enthält 77—82% Phenole, 18—22% Wasser, 0,5—2,0% Phenolat und 3—5% Soda.

Beide Phasen werden getrennt.

Die organische Phase (ca. 200—220 kg) wird mit Diisopropyläther (80 kg) vermischt, wonach sich aus dem Gemisch ca. 15 kg Sodalösung abscheiden. Die Sodalösung wird abgetrennt und zur primär angefallenen Soda gegeben.

Das Äther-Phenol-Wasser-Gemisch wird dann mit 15—20 Gew.-% Wasser im Gegenstrom weitgehend salzfrei gewaschen (>0,09% Alkali).

Das Waschwasser enthält Soda und Natriumphenolat (6% Phenole und 3—5% Alkali) und wird zur primär angefallenen Sodalösung gegeben, wo das Natriumphenolat mit Bicarbonat zu Soda und Phenol weiter umgesetzt wird.

Aus dem Äther-Phenol-Wasser-Gemisch werden Äther und Wasser destillativ entfernt. Hierbei werden ca. 210 kg Rohphenol (wasserhaltig) gewonnen. Bei der folgenden Destillation kann das Rohphenol wegen des geringen Salzgehaltes bis auf 2—4% ausdestilliert werden.

Der aus der Destillation gewonnene Äther (77—79 kg) wird eingesetzt, um das Gemisch aus primärer und sekundärer Soda sowie dem Waschwasser durch Extraktion zu entphenolen.

Nach der Entphenolung enthält die Soda 20—30 ppm Phenol. Somit ist der Äther-Kreislauf geschlossen. Die Verluste an Diisopropyläther liegen bei 1—2%.

Das bei der Destillation abgetriebene Wasser wird zur Extraktion des Äther-Rohphenol-Wasser-Gemisches wieder verwendet.

### Patentanspruch

Verfahren zur Aufbereitung von durch Fällung mit Kohlendioxid aus Natriumphenolat erzeugtem Rohphenol durch Extraktion der phenolhaltigen Sodalauge und des abgetrennten Rohphenols mit Lösungsmittel, dadurch gekennzeichnet, daß kontinuierlich der phenolhaltige Lösungsmittelextrakt aus der Sodalaugeextraktion mit Rohphenol gemischt, das Gemisch mit Wasser im Gegenstrom gewaschen und das Waschwasser, das Phenolat, Soda und Bicarbonat enthält, zur phenolhaltigen Sodalauge zurückgegeben wird.

### Claim

A process for the treatment of raw phenol, produced by reaction of sodium phenolate with carbon dioxide, whereas the phenol containing soda lye and the separated raw phenol are extracted with a solvent, characterized in that continuously the phenol containing solvent extract from the soda lye extraction is mixed with raw phenol, the mixture is washed with water in counter current flow and the wash water, which contains phenolate, soda and bicarbonate, is returned to the phenol containing soda lye.

### Revendication

Procédé de traitement de phénol brut obtenu par précipitation de phénolate de sodium avec du dioxyde de carbone, lequel phénol brut provient de l'extraction de la liqueur de carbonate de sodium contenant du phénol et de la séparation du phénol brut avec un solvant, caractérisé en ce qu'on mélange en continu, avec le phénol brut, l'extrait de solvant contenant du phénol obtenu à partir de l'extraction de la liqueur de carbonate de sodium, lequel mélange est lavé avec de l'eau à contre-courant et contient de l'eau de lavage, le phénolate, le carbonate de sodium et le bicarbonate, pour être recyclé dans la liqueur de carbonate de sodium contenant le phénol.